# EUROPEAN PATENT APPLICATION

(11) **EP 1 967 192 A1**
(43) Date of publication of application: **10.09.2008**
(21) Application number: 08250772.4
(22) Date of filing: 07.03.2008
(51) Int. Cl.: A61K 31/4965, A61P 25/28

(54) **N-amidino pyrazinecarboxamide derivatives for use in the prophylaxis or treatment of neurodegenerative diseases**

(30) Priority: 09.03.2007 JP 2007060829
(71) Applicant: Riken, Wako-shi, Saitama 351-0198 (JP)
(72) Inventor: Nukina, Nobuyuki, Wako-shi, Saitama 351-0198 (JP); Wong, Hon-kit, Wako-shi, Saitama 351-0198 (JP)
(74) Representative: Duckworth, Timothy John

(57) **Abstract**

The present invention provides a novel and useful agent for the prophylaxis or treatment of neurodegenerative diseases. Particularly, the present invention provides an agent for the prophylaxis or treatment of a neurodegenerative disease, which contains a compound represented by the following formula (I) as an active ingredient: 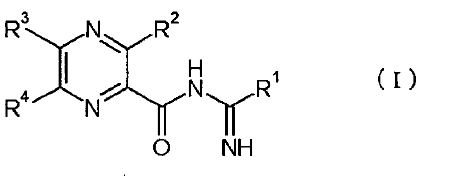 wherein R¹ , R², R³ are each independently an optionally substituted amino group, and R⁴ is halogen.

SEQUENCE LISTING

<110>  Riken

<120>  AGENT FOR THE PROPHYLAXIS OR TREATMENT OF NEURODEGENERATIVE
       DISEASE

<130>  N.104287

<150>  JP 060829/2007
<151>  2007-03-09

<160>  34

<170>  PatentIn version 3.4

<210>  1
<211>  20
<212>  DNA
<213>  Artificial

<220>
<223>  NHE1 forward primer

<400>  1
tacggggtca tcgcggcttt                                                   20


<210>  2
<211>  20
<212>  DNA
<213>  Artificial

<220>
<223>  NHE1 reverse primer

<400>  2
atgacggtga tgatggcggt                                                   20


<210>  3
<211>  20
<212>  DNA
<213>  Artificial

<220>
<223>  NHE5 forward primer

<400>  3
ccacagaaga acccacccag                                                   20


<210>  4
<211>  20
<212>  DNA
<213>  Artificial

<220>
<223>  NHE5 reverse primer

<400>  4
aaagacggcc aacacggcca                                                    20


<210>  5
<211>  20
<212>  DNA
<213>  Artificial

<220>
<223>  NCX1 forward primer

<400>  5
tgaccggagc tggcaacatc                                                    20


<210>  6
<211>  20
<212>  DNA
<213>  Artificial

<220>
<223>  NCX1 reverse primer

<400>  6
tctcgctcac gtgagtcacg                                                    20


<210>  7
<211>  20
<212>  DNA
<213>  Artificial

<220>
<223>  NCX2 forward primer

<400>  7
aggctgccaa ggttcctacc                                                    20


<210>  8
<211>  20
<212>  DNA
<213>  Artificial

<220>
<223>  NCX2 reverse primer

<400>  8
cccacacctg gactacacct                                                    20


<210>  9
<211>  20
<212>  DNA
<213>  Artificial

<220>
<223>  NCX3 forward primer

<400>  9
cttcgtcacg gctgcttgga                                                    20


<210>  10
<211>  20
<212>  DNA
<213>  Artificial

<220>
<223>  NCX3 reverse primer

<400>  10
ctcaatgccc ttagggtggt                                                    20


<210>  11
<211>  20
<212>  DNA
<213>  Artificial

<220>
<223>  BNaC1 forward primer

<400>  11
ccgtccctga gtcgcactaa                                                    20


<210>  12
<211>  20
<212>  DNA
<213>  Artificial

<220>
<223>  BNaC1 reverse primer

<400>  12
catgaaggca gcgctgatgc                                                    20


<210>  13
<211>  20
<212>  DNA
<213>  Artificial

<220>
<223>  BNaC2 forward primer

<400>  13
tgcacggtct tgcccacatc                                                    20


<210>  14
<211>  20
<212>  DNA
<213>  Artificial

<220>
<223>  BNaC2 reverse primer

<400>  14
gtcatgccct gctctgtcgt                                                    20


<210>  15
<211>  20
<212>  DNA
<213>  Artificial

<220>
<223>  egfp forward primer

<400>  15
atggtgagca agggcgagga                                                    20


<210>  16
<211>  20
<212>  DNA
<213>  Artificial

<220>
<223>  egfp reverse primer

<400>  16
catgccgaga gtgatcccgg                                                    20


<210>  17
<211>  20
<212>  DNA
<213>  Artificial

<220>
<223>  Gapdh forward primer

<400>  17
attgtcagca atgcatcctg                                                    20


<210>  18
<211>  23
<212>  DNA
<213>  Artificial

<220>
<223>  Gapdh reverse primer

<400>  18
ttcagctctg ggatgacctt gcc                                                23


<210>  19
<211>  19
<212>  DNA
<213>  Artificial

<220>
<223>  NHE1-1 shRNA producing sequence

<400>  19
ggacaagctc aaccgtttt                                                    19


<210>  20
<211>  19
<212>  DNA
<213>  Artificial

<220>
<223>  NHE1-2 shRNA producing sequence

<400>  20
ggaggaagag atccgcaaa                                                    19


<210>  21
<211>  19
<212>  DNA
<213>  Artificial

<220>
<223>  NHE5-1 shRNA producing sequence

<400>  21
gaggagtgat taccacaaa                                                    19


<210>  22
<211>  19
<212>  DNA
<213>  Artificial

<220>
<223>  NHE5-2 shRNA producing sequence

<400>  22
agggcgtcgc ctccctgtt                                                    19


<210>  23
<211>  19
<212>  DNA
<213>  Artificial

<220>
<223>  NCX1-1 shRNA producing sequence

<400>  23
ctgggtctga ttatgaatt                                                    19


<210>  24
<211>  19
<212>  DNA
<213>  Artificial

<220>
<223>  NCX1-2 shRNA producing sequence

<400>  24
agagttggca tcattgatg                                                    19


<210>  25
<211>  19
<212>  DNA
<213>  Artificial

<220>
<223>  NCX2-1 shRNA producing sequence

<400>  25
cttcaggtca agatagtgg                                                    19


<210>  26
<211>  19
<212>  DNA
<213>  Artificial

<220>
<223>  NCX2-2 shRNA producing sequence

<400>  26
gaatggagac aagaagata                                                    19


<210>  27
<211>  19
<212>  DNA
<213>  Artificial

<220>
<223>  NCX3-1 shRNA producing sequence

<400>  27
taattgatga taaggcgta                                                    19


<210>  28
<211>  19
<212>  DNA
<213>  Artificial

<220>
<223>  NCX3-2 shRNA producing sequence

<400>  28
acaattcggg tatggaatg                                                    19


<210>  29
<211>  19
<212>  DNA
<213>  Artificial

<220>
<223>  BNaC1/ASIC2-1 shRNA producing sequence

<400>  29
acagcaatga acaccaaag                                                    19


<210>  30
<211>  19
<212>  DNA
<213>  Artificial

<220>
<223>  BNaC1/ASIC2-2 shRNA producing sequence

<400>  30
cccaaggaca gcaatgaac                                                    19


<210>  31
<211>  19
<212>  DNA
<213>  Artificial

<220>
<223>  BNaC2/ASIC1-1 shRNA producing sequence

<400>  31
tcaatgagtt tcgctttag                                                    19


<210>  32
<211>  19
<212>  DNA
<213>  Artificial

<220>
<223>  BNaC2/ASIC1-2 shRNA producing sequence

<400>  32
gagcgtgtgc agtactact                                                    19


<210>  33
<211>  19
<212>  DNA
<213>  Artificial

<220>
<223>  BNaC2/ASIC1-3 shRNA producing sequence

<400>  33
agttcaacaa atctgaaca                                                    19


<210>  34
<211>  19
<212>  DNA
<213>  Artificial

<220>
<223>  LacZ shRNA producing sequence

<400> 34
tctatcgtgc ggtggttga 19

## Description

### Technical Field of the Invention

The present invention relates to an agent for the prophylaxis or treatment of neurodegenerative diseases.

### Background of the Invention

As one of the neurodegenerative diseases, polyglutamine disease is known wherein abnormal expansion of CAG base sequence repeat in a responsible gene leads to abnormal accumulation of a responsible gene product containing an abnormally expanded glutamine chain in the neurons, which triggers neuronal cell death and dysfunction.

As the polyglutamine disease, Huntington's disease, spinal and bulbar muscular atrophy, spinocerebellar ataxia type I, spinocerebellar ataxia type II, spinocerebellar ataxia type III (Machado-Joseph disease), spinocerebellar ataxia type VI, spinocerebellar ataxia type VII, spinocerebellar ataxia type XVII, dentatorubropallidoluysian atrophy and the like are known (Ross C.A., Neuron 35, 819-822 (2002)).

Of these diseases, Huntington's disease is an autosomal dominantly-inherited progressive fatal neurodegenerative disease. Its morbidity rate is about 5 people out of 100000 (0.005%) worldwide, and the median age of onset is said to be 39 years old.

Huntington's disease is characterized by motor symptoms (chorea, dystonia, incoordination, postural instability), cognitive symptoms (deficits in short term memory, skill learning, planning and organization), and emotional symptom (depression).

The clinical manifestation of Huntington's disease is found when the number of CAG repeats in exon 1 of Huntington's disease gene exceeds 35. The onset of Huntington's disease in adult is generally correlated with polyglutamine (polyQ) repeat length of 40 - 60, and that in young people is generally correlated with polyQ repeat length exceeding 70 repeats.

When abnormally expanded polyglutamine is expressed, abnormal aggregate is formed in the neurons. While the chaperone system tries to suppress the aggregation, when the aggregation is not successfully suppressed, the cellular proteasome proteolytic system is inhibited, which consequently causes neuronal cell death (Wang, G.H., Mitsui, K., Kotliarova, S., Yamashita, A., Nagao, Y., Tokuhiro, S., Iwatsubo, T., Kanazawa, I., and Nukina, N., Neuroreport 10, 2435-2438 (1999); Jana, N.R., Tanaka, M., Wang, G. and Nukina, N., Hum Mol Genet 9, 2009-2018 (2000); Jana, N.R., Zemskov, E.A., Wang, G., and Nukina, N., Hum Mol Genet 10, 1049-1059 (2001)).

Amiloride is a known compound used as a potassium-sparing distal diuretic that expresses its action by inhibiting the sodium channel.

Benzamil and phenamil, which are amiloride analogs, are also known as sodium channel inhibitors, and these compounds are known to be usable for the treatment of lung disease (US Patent No. 5,656,256).

### Disclosure of the Invention

While agents for the prophylaxis or treatment of neurodegenerative diseases have been developed from various aspects, a novel and useful prophylactic or therapeutic agent has still been demanded since fatal diseases are included.

We have found that amiloride and analog thereof used as therapeutic agents for renal diseases and lung diseases are unexpectedly useful for the neurodegenerative diseases, particularly, those caused by abnormality in the degradation mechanism of abnormal protein as represented by abnormal expansion of CAG repeats and the like, which resulted in the completion of the present invention.

The present invention provides the following:
[1] A use of a compound represented by the formula (I) 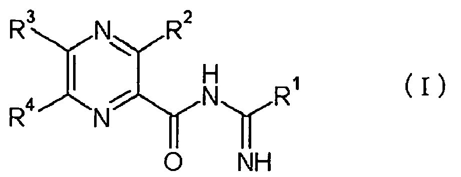 wherein R¹, R² and R³ are each independently an optionally substituted amino group, and R⁴ is halogen, or a pharmacologically acceptable salt thereof for the production of an agent for the prophylaxis or treatment of a neurodegenerative disease.
[2] The use of the above-mentioned [1], wherein R¹ is an amino group substituted by an optionally substituted lower alkyl group or an optionally substituted aryl group.
[3] The use of the above-mentioned [2], wherein R¹ is an amino group substituted by an unsubstituted aryl group or a lower alkyl group substituted by an aryl group, and R² and R³ are each an unsubstituted amino group.
[4] The use of any of the above-mentioned [1] to [3], wherein R⁴ is chlorine.
[5] The use of the above-mentioned [1], wherein the compound represented by the formula (I) is amiloride, benzamil or phenamil.
[6] The use of any of the above-mentioned [1] to [5], wherein the neurodegenerative disease is selected from the group consisting of polyglutamine disease, Alzheimer's disease, Parkinson's disease and amyotropic lateral sclerosis.
[7] The use of any of the above-mentioned [1] to [5], wherein the neurodegenerative disease is Huntington's disease.
[8] A use of a compound represented by the formula (I) of the above-mentioned [1] or a pharmacologically acceptable salt thereof for the production of a proteasome activity enhancer.
[9] A compound represented by the following formula (I) of the above-mentioned [1] or a pharmacologically acceptable salt thereof for use in a method for the prophylaxis or treatment of a neurodegenerative disease.
[10] A compound represented by the formula (I) of the above-mentioned [1] or a pharmacologically acceptable salt thereof for use in a method for enhancing a proteasome activity.

According to the present invention, a novel agent for the prophylaxis or treatment of a neurodegenerative disease can be provided.

### Brief Description of the Drawings

Fig. 1. The expression level of genes was significantly blocked at both the transcriptional level (a) and the post-translational level (b, c) by the corresponding shRNAs. (c) is quantitative data of the experiment of (b) (showing only for NHE1, BnaC2) (N1=NHE1, X1 - 3=NCX1 - 3, B1 - 2=BnaC1 - 2).
Fig. 2. Amiloride and a derivative thereof benzamil, decreased tNhtt-polyQ aggregation through a post-transcriptional mechanism in a dose-dependent manner.
   (a) A Neuro2a cell line was induced for 1 day so as to form tNhtt-60Q or tNhtt-150Q, and the number of EGFP-positive aggregates was quantified by the ArrayScan. The addition of amiloride or benzamil decreased the number of aggregates in a dose-dependent manner. n=5, * and **p=0.019;*** and ****p<0. 0001.
   (b) The addition of benzamil up to 50 µM did not cause significant toxicity against Neuro2a cells. Neuro2a cells treated with benzamil of the indicated various concentrations were stained with 5 µg/ml proprium iodide (PI) at 37°C for 10 min to detect cell death. n=3, *p=0.105 (N.S., not significant); **p=0.001.
   (c) Amiloride and benzamil decreased nuclear aggregates formed by tNhtt-150Qnls as quantified by the ArrayScan. n=3, * and **p=0.03.
   (d) Benzamil decreased aggregates formed by tNhtt-polyQ (+/nls) in the transient expression system. Cells were transiently transfected for 5 hr with the indicated plasmid expressing various length of polyQ (+/- nls), and then 50 µM of benzamil was added. 12 hr later, cells were collected for quantification by the ArrayScan. n=3, *p<0.0001.
   (e) Amiloride and benzamil decreased the aggregate load shown by a filter trap assay. After the indicated treatment, cells were lysed, and the lysates were treated with 2% SDS. Both amiloride and benzamil decreased SDS-resistant aggregates trapped on a membrane. β-Tubulin shows equal protein loading. (f, g) Semi-quantitative PCR showed unaltered expression of tNhtt-polyQ in cells treated with amiloride and benzamil of various concentrations. The expression level of the transgene was amplified using primers targeting EGFP or tNhtt-poly-EGFP. Gapdh shows equal RNA input. Amiloride or benzamil up to 50 µM did not apparently alter the expression level of the transgene shown by 2 different primer pairs suggesting that the removal of aggregates is mediated by the post-transcriptional mechanism. The quantification of the PCR result for tNhtt-16Q is shown in (g).

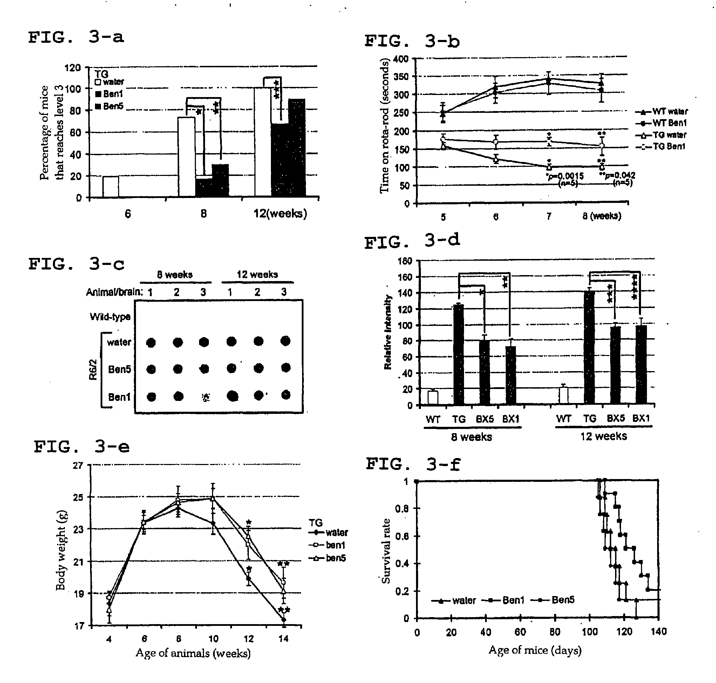

Fig. 3. Benzamil ameliorated the motor deficits and pathology, and improved the survival rate in R6/2 mice.
   (a) Clasping score. The benzamil treatment remarkably decreased the number of mice reaching clasping score level 3 in both 8 week-old mice and 12 week-old mice. For grading, see the description provided in the Examples. n=10 in each group. * and **p<0.0001 and ***p=0.009.
   (b) Rota-rod test. Benzamil in an amount of 1 mg/kg alleviated the motor deficit of R6/2 mice by improving the performance on the rotating rod. n=5 in each group. *p=0.0015 and **p=0.042.
   (c, d) The benzamil treatment decreased the amount of SDS-resistant aggregates demonstrated with a filter trap assay. The SDS-treated lysates collected from 3 individual animals of each age (8 or 12 weeks) were each applied onto a cellulose acetate membrane. In brain lysates from the benzamil-treated (1 mg/kg and 5 mg/kg) mouse, aggregates trapped on the membrane were less. (d) shows the quantification results of (c). n=3, *p=0. 009; **p=0. 004; *** and ****p=0.007.
   (e) The benzamil treatment increased the body weight of R6/2 mice. The benzamil treatment increased the body weight of R6/2 mice after 8 week-old. Tg-water, n=10; tg-Ben1, n=13 and tg-Ben5, n=10. *p=0.034 and **p=0.044.
   (f) Benzamil significantly increased the survival rate of R6/2 mice. The survival data from each treatment were analyzed by the Kaplan-Meier method followed by log-rank testing. The average survival period for tg-water was 114.625±2.44 days, and that for tg-Ben1 was 126.1±4.001 days (tg-water vs tg-Ben1, p=0.024). The data are presented as average ± S.E.M.

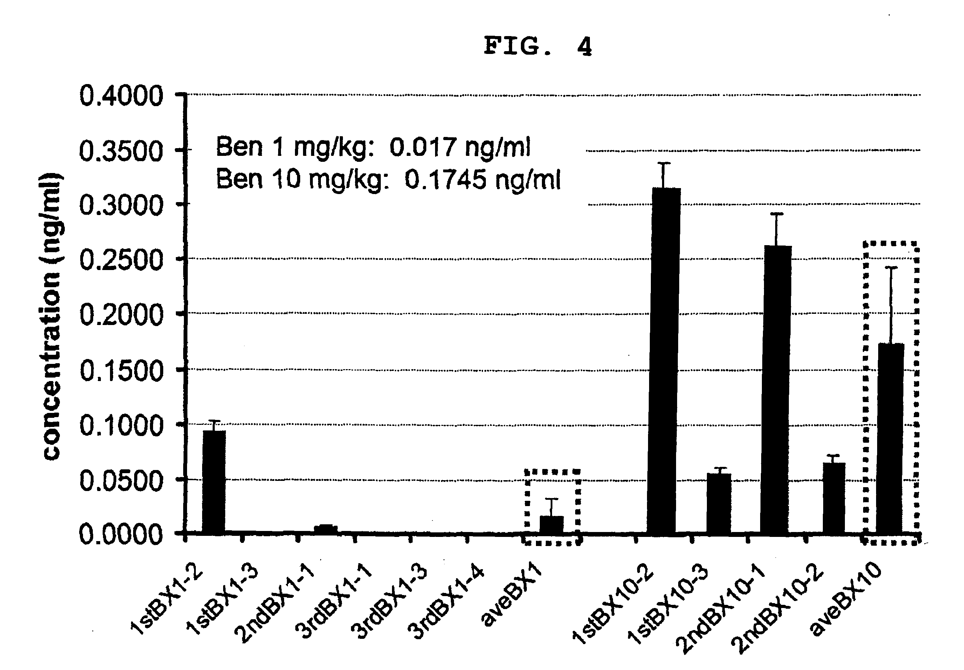

Fig. 4. Since benzamil could be detected and quantified in the cerebrospinal fluid (CSF) of the experimental rats, it is believed that benzamil exerts a direct effect in the brain tissue to decrease the amount of polyQ aggregates.

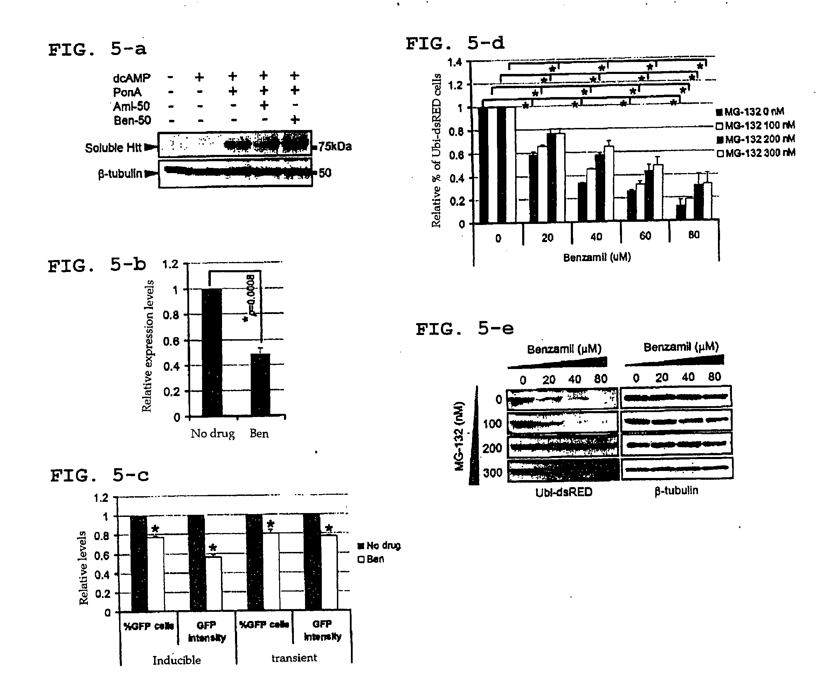

Fig. 5. The expression level of soluble tNhtt-polyQ was decreased by the benzamil treatment, and benzamil enhanced *in vivo* proteasome activity.
   (a, b) The addition of amiloride or benzamil decreased the expression level of soluble tNhtt-150Q shown by a Western blot. The cells treated with amiloride or benzamil were lysed and subjected to a Western blot with an anti-huntingtin antibody. Bothe amiloride and benzamil treatment decreased the expression level of soluble tNhtt-150Q. β-Tubulin shows protein loading. The quantification of tNhtt-150Q expression after the benzamil treatment is shown in (b). n=3, *p=0.0008.
   (c) The ArrayScan revealed that the expression of soluble tNhtt-150Q in benzamil-treated cells was decreased, in both the inducible and transient transfection systems. n=5, *p<0.0001 (compared with the water control).
   (d, e) Benzamil alleviated the proteasome inhibition caused by MG-132 in a dose-dependent manner (d, ArrayScan quantification of ubi-dsRED cells; e, Western blot analysis with anti-RFP antibody). Neuro2a cells were first transfected with an ubi-dsRED construct for 5 hr, and then treated with increasing concentration of MG-132 to block UPS. The UPS blocking increased the accumulation of ubi-dsRED in a concentration-dependent manner. On the other hand, benzamil alleviated the ubi-dsRED accumulation. This indicates that benzamil can globally enhance UPS activity. β-Tubulin shows the equal protein loading in (e). n=3, *p=0.019.

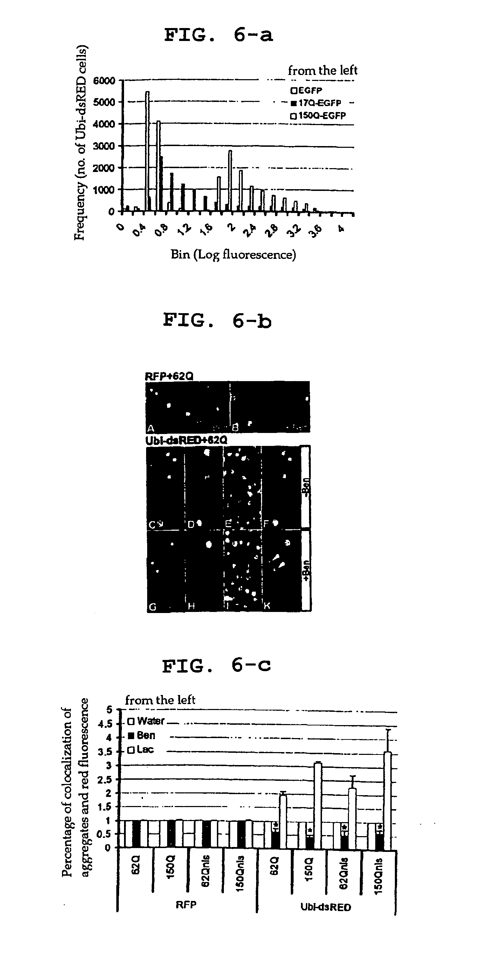

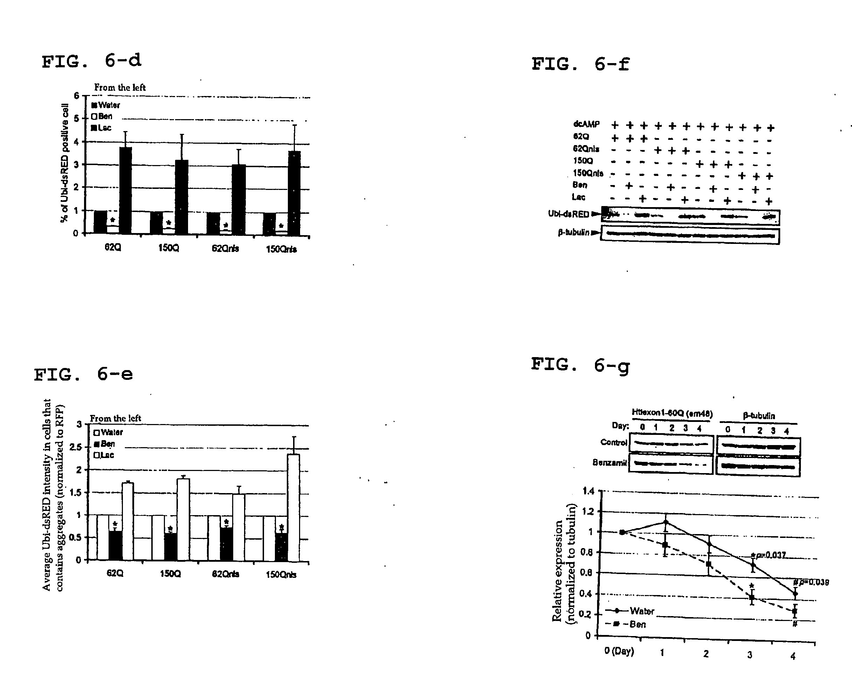

Fig. 6. Benzamil enhanced the proteasome activity in tNhtt-polyQ-bearing cells and promoted the degradation of tNhtt-60Q.
   (a) The proteasome activity was blocked in a polyQ length-dependent manner. Neuro2a cells were co-transfected with ubi-dsRED and EGFP expressing (EGFP only, tNhtt-17Q-EGFP or tNhtt-150Q-EGFP) constructs, and 18 hr later, the intensity of ubi-dsRED fluorescence was analyzed by the ArrayScan. The EGFP transfection gave relatively weak signals, but tNhtt-150Q-EGFP generated strong signals. This indicates that UPS is blocked by glutamine repeats in a polyQ length-dependent manner. This experiment demonstrated that ubi-dsRED was an effective tool to show the UPS activity in polyQ-bearing Neuro2a cells. Each population contained more than 10,000 cells, and distribution between different populations was highly significant. All p<0.0001.
   (b, c) Benzamil attenuated the UPS blocking caused by tNhtt-polyQ. A fluorescence microscopic image shows co-expression of RFP- and EGFP-positive 62Q aggregates (A and B) and co-expression of ubi-dsRED and 62Q (C - K) in the presence or absence of benzamil. The addition of benzamil alleviated UPS blocking globally such that the UPS activity of polyQ-bearing cells is enhanced. As a result, the co-localization of ubi-dsRED and polyQ was decreased, and on the other hand, the co-localization of RFP and 62Q was substantially unaltered (c: quantified by the ArrayScan, *p<0.0001). Lactacystin served as a positive control for UPS blocking.
   (d, e, f) Additional evidence showing that benzamil enhances UPS activity in polyQ-bearing cells. Benzamil decreased the percentage of ubi-dsRED cells (d) and the average intensity of ubi-dsRED (e) in cells carrying visible polyQ aggregates both in the cytoplasm and nucleus, quantified by the ArrayScan. By a Western blot analysis with an anti-RFP antibody using cells from the same experiment setting, data obtained by the ArrayScan were confirmed (f). Lactacystin had ubi-dsRED accumulated while benzamil attenuated ubi-dsRED. n=3, *p<0.0001.
   (g) Benzamil enhanced the degradation of soluble tNhtt-60Q revealed by a chase experiment. With the addition of benzamil, the degradation of soluble tNhtt-60Q was enhanced, and the absolute amount of soluble tNhtt-60Q was significantly less after 3 and 4 days of the benzamil treatment. n=3, *p=0.037 and #p=0.039.

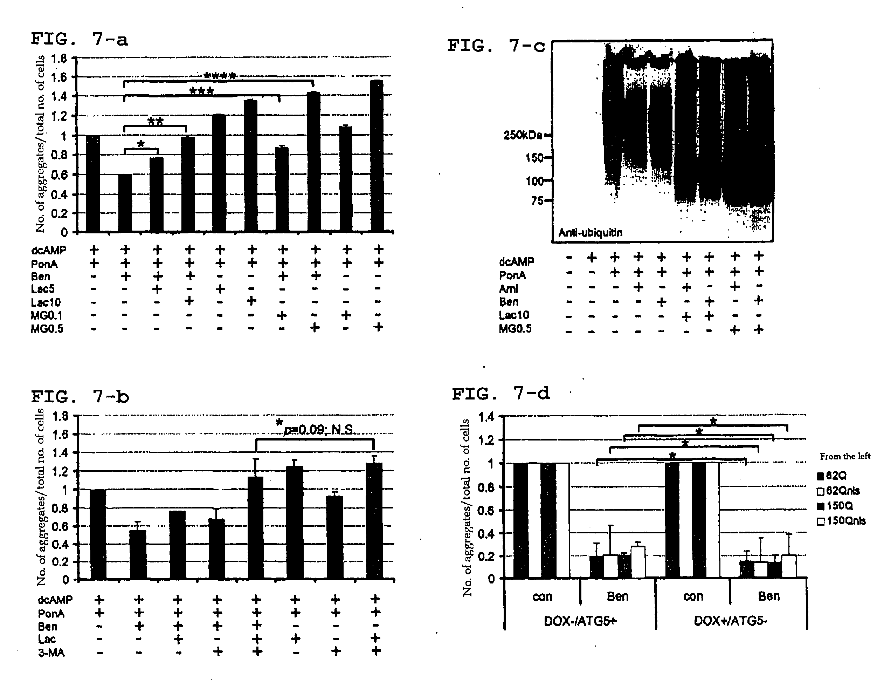

Fig. 7. UPS, but not autophagy, is the major pathway through which benzamil enhances tNhtt-polyQ clearance.
   (a) The UPS blocking remarkably abolished the effect of benzamil. In addition to the ponasterone A induction of tNhtt-150Q, Neuro2a cells were incubated with benzamil and proteasome inhibitor, i.e., lactacystin or MG-132, at two different concentrations. Lactacystin or MG-132 was also used as a control in the absence of benzamil. Lactacystin or MG-132 remarkably reversed and abolished the disaggregating effect of benzamil. This indicates that UPS is the degradation system mainly employed by benzamil for tNhtt-polyQ clearance. n=3, *p=0.006;**, *** and ****p<0.0001.
   (b) Blocking both UPS and macroautophagy completely abolished the effect of benzamil. When 5 mM 3-methyladenine (3-MA) was added together with 5 µM lactacystin, the disaggregating effect of benzamil was completely abolished. This suggests a possibility that macroautophagy may also be involved in the degradation of tNhtt-polyQ, although its involvement may not be very significant. n=3, *p=0.09, N.S., not significant.
   (c) Consistent with the results obtained in (a), a Western blot analysis with an anti-ubiquitin antibody also revealed that proteasome inhibition abolished the disaggregating effect of benzamil.
   (d) Benzamil decreased tNhtt-polyQ aggregation in the absence of Atg5 (essential component of macroautophagy). MEF was incubated with 1 ng/ml doxycycline for 5 days, and polyQ of various lengths (+/- nls) was transiently transfected. The ability of benzamil to decrease aggregation to the same extent in the presence or absence of Atg5 indicates that macroautophagy is not required for the tNhtt-polyQ clearance caused by benzamil, further supporting the data obtained in (a) - (c). n=3, *p>0.05, not significant.

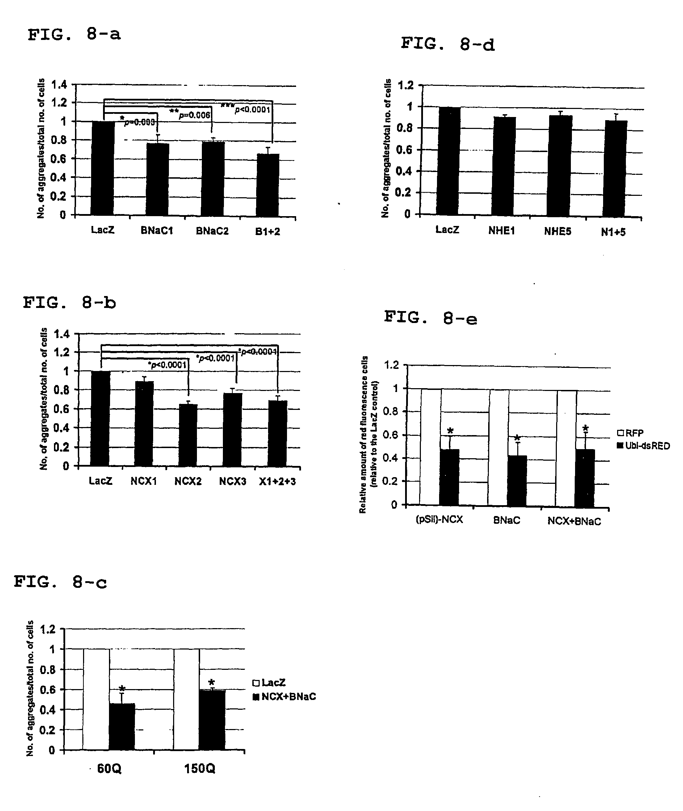

Fig. 8. Silencing of ASIC/BNaC or NCX expression decreased tNhtt-polyQ aggregation and increased *in vivo* proteasome activity.
   (a) Silencing of ASIC/BNaC expression decreased the amount of tNhtt-150Q aggregation. Neuro2a cells were first transiently transfected for 2 days with psilencer 1.0-U6 encoding shRNA specific for BNaC silencing, and then truncated Nhtt-150Q was induced for 1 day. LacZ shRNA was used as a negative control for transfection and silencing. A decrease of the expression level of BNaC1 (ASIC2), BNaC2 (ASIC1) or both led to a decrease of tNhtt-150Q aggregation. This suggests that the expression or activity of these protein channels may be involved in the formation or progression of tNhtt-polyQ aggregation. n=9, *p=0.003;**p=0.006 and ***p<0.0001.
   (b) Silencing of NCX expression decreased the amount of tNhtt-polyQ aggregation. n=9, *p<0.0001.
   (c) Silencing of both BNaC and NCX expressions decreased the amount of tNhtt-60Q and tNhtt-150Q aggregates. n=5, *p<0.0001.
   (d) Silencing of NHE expression did not alter the amount of tNhtt-150Q aggregates.
   (e) Silencing of the expression of NCX, BNaC or both channel proteins led to significant enhancement of proteasome activity. Neuro2a cells were first transfected with the corresponding psilencer shRNAs for 2 days, thereby silencing the gene. Subsequently, a 70 ng ubi-dsRED construct was transfected to evaluate the *in vivo* proteasome activity under these conditions. Although there was no significant change in RFP expression (the same percentage of RFP cells in the whole population), ubi-dsRED expression was remarkably decreased compared with the LacZ shRNA control. n=3, *p<0.0001.

### Best Mode for Embodying the Invention

The agent for the prophylaxis or treatment of neurodegenerative diseases, which is provided by the present invention (hereinafter to be also referred to as a prophylactic or therapeutic agent of the present invention), contains a compound represented by the following formula (I) or a pharmacologically acceptable salt thereof as an active ingredient. 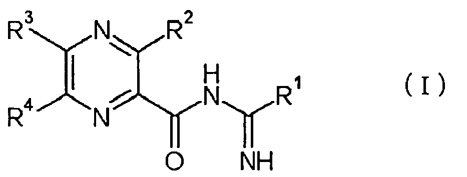 wherein R¹, R² and R³ are each independently an optionally substituted amino group, and R⁴ is halogen.

The substituent that the amino group for R¹, R² or R³ may have may be any as long as it does not adversely influence the prophylactic or therapeutic effect of a compound represented by the formula (I) on neurodegenerative diseases and, for example, an optionally substituted lower alkyl group, an optionally substituted aryl group and the like can be mentioned, with preference given to an optionally substituted lower alkyl group and an optionally substituted aryl group.

Examples of the substituent of the above-mentioned lower alkyl group include halogen, a substituted or unsubstituted aryl group and the like, and preferably an unsubstituted aryl group.

In the present specification, the lower alkyl group is a straight chain or branched chain alkyl group having 1 to 6 carbon atoms. Examples thereof include methyl, ethyl, propyl, isopropyl, butyl, isobutyl, tert-butyl, pentyl, hexyl and the like. Preferred is straight chain alkyl group having 1 to 6 carbon atoms, and particularly, methyl.

The aryl group in the present specification may have substituent(s) such as halogen, optionally substituted lower alkyl group, optionally substituted aryl group and the like, preferably unsubstituted aryl group such as phenyl, naphthyl and the like, and particularly preferably phenyl, at substitutable position(s).

Examples of the halogen in the specification include fluorine, chlorine, bromine and the like.

As the halogen for R⁴, fluorine or chlorine is preferable, and chlorine is particularly preferable.

As the compound represented by the formula (I), amiloride, benzamil and phenamil are particularly preferable.

The target neurodegenerative diseases for the prophylactic or therapeutic agent of the present invention are diseases that can be prevented or treated by enhancing the proteasome activity, such as polyglutamine disease,
Alzheimer's disease, Parkinson's disease, amyotrophic lateral sclerosis and the like, and polyglutamine disease is particularly preferable.

The polyglutamine disease is not particularly limited as long as it is characterized by abnormal expansion of CAG repeats. For example, Huntington's disease, spinal and bulbar muscular atrophy, spinocerebellar ataxia type I, spinocerebellar ataxia type II, spinocerebellar ataxia type III (Machado-Joseph disease), spinocerebellar ataxia type VI, spinocerebellar ataxia type VII, spinocerebellar ataxia type XVII, dentatorubropallidoluysian atrophy and the like can be mentioned.

As used herein, the abnormal expansion of CAG repeats means that the responsible gene contains not less than 36 CAG repeats, and preferably not less than 40 CAG repeats.

The present invention also provides a proteasome activity enhancer containing a compound represented by the formula (I) or a pharmacologically acceptable salt thereof as an active ingredient (hereinafter to be also referred to as an enhancer of the present invention). Since a compound represented by the formula (I) and a pharmacologically acceptable salt thereof act on a channeling molecule and enhance the proteasome activity, they can promote proteolysis by proteasome and suppress protein aggregation caused by abnormal folding of protein. In addition, they can suppress neuronal cell death caused by abnormal folding and protein aggregation.

Where necessary, a compound represented by the formula (I) may be converted to a pharmacologically acceptable salt, hydrate or solvate thereof. Examples of the pharmacologically acceptable salt include salts with inorganic acid (hydrochloride, hydrobromide, phosphate, sulfate and the like), salts with organic acid (acetate, succinate, maleate, fumarate, malate, tartrate and the like) and the like.

The prophylactic or therapeutic agent and enhancer of the present invention can be administered orally or parenterally as it is or mixed the agent with a pharmacologically acceptable carrier and the like, in a dosage form of, for example, a solid preparation such as powder, granule, tablet, capsule and the like, liquid such as syrup, emulsion, injection (including subcutaneous injection, intravenous injection, intramuscular injection, drip infusion) and the like, sublingual tablet, buccal preparation, troche, microencapsulated or sustained-release coated preparation, suppository and the like.

As the pharmacologically acceptable carrier, various organic or inorganic carriers conventionally used as preparation materials can be used. For a solid dosage form, excipient, lubricant, binder, disintegrant and the like are appropriately used and, for a liquid preparation, solvent, solubilizing agent, suspending agent, isotonicity agent, buffering agent, soothing agent and the like are appropriately used. Where necessary, various additives such as preservative, antioxidant, colorant, sweetening agent, flavor and the like may be added.

A preparation in the above-mentioned dosage form can be produced according to a formulation method known in the field.

Subjects for administration of the prophylactic or therapeutic agent and enhancer of the present invention include, for example, mammals such as mouse, rat, hamster, rabbit, cat, dog, bovine, sheep, monkey, human and the like.

The present invention is explained in more detail in the following by referring to Examples, which are not to be construed as limitative.

### Examples

### Reagent and antibody

Amiloride·HCl and benzamil·HCl were obtained from Alexis; lactacystin was obtained from Peptide Institute, Inc.; MG-132 (Z-Leu-Leu-Leu-aldehyde) was obtained from Wako Pure Chemical Industries, Ltd.; an anti-huntingtin antibody and an anti-β-tubulin antibody were obtained from Chemicon; an anti-RFP antibody was obtained from MBL, and an anti-V5 antibody was obtained from Invitrogen. Unless stated otherwise, other chemicals were all obtained from Sigma.

### Cell culture and treatment

In a 5% CO₂ incubator at 37°C, neuroblastoma 2a (Neuro2a) cells and mouse embryonic fibroblasts (MEFs) were maintained in DMEM supplemented with 10% inactivated FBS (Sigma), 2 mM L-glutamine, 0.4 mg/ml Zeocin, 0.4 mg/ml G418 (InvivoGen) and 100 U/ml penicillin/100 µg/ml streptomycin (GIBCO™ Invitrogen). Neuro2a cells were induced to express tNhtt-polyQ with 1 µM ponasterone A and differentiated into neuronal phenotype by 5 mM dbcAMP. Amiloride or benzamil was incubated with inducible Neuro2a cells at the time of differentiation and induction. For the transient expression of tNhtt-polyQ, in order to avoid interference to transfection efficiency, the test drug was introduced 5 hours after transfection.

### Expression construct

The mRNA sequence of a channel protein was cloned into a pcDNA™3.1/V5-His TOPO® TA expression vector (Invitrogen) according to the instruction manual. The full-length mRNA sequence of each gene was amplified by nested PCR. The accession number of each mRNA is as follows: NHE-1, U51112; NHE-5, AF111173; NCX-1, NM_011406; NCX-2, NM_148946; NCX-3, NM_080440; BNaC1, BC038551; and BNaC2, NM_009597. All the plasmids were sequence-verified, and the expression and expected molecular weight thereof were checked by Western blot with an anti-V5 antibody (Invitrogen). In order to express the truncated N-terminal of human huntingtin having 17, 62 or 150 glutamine repeats fused to enhanced GFP, constructs each encoding the fused protein were prepared using pEGFP-N1 as described in Wang, G.H. et al. Neuroreport 10, 2435-8 (1999). The cDNA of monomeric fluorescent protein (mRFP) was derived from the mRFP1 in pRSET_{B} and ligated into pcDNA3 as described in Machida, Y. et al., Biochem Biophys Res Commun 343, 190-7 (2006). The ubiquitinated (ubi) Discosoma Red fluorescent protein (dsRed)2/N1 plasmid was prepared and used as described in Khan, L.A. et al. J Neurochem 98, 576-87 (2006).

### DNA transfection

DNA was transiently transfected into the Neuro2a cells using Lipofectamine 2000 (Invitrogen) according to the instruction manual. Generally, for the transient expression of tNhtt-polyQ and ubi-dsRED (or RFP), DNA was transfected in amounts of 200 ng and 70 ng, respectively. Cells were transfected for 5 hr, and the media were replaced for the subsequent treatment.

### ArrayScan® quantification

To quantify the percentage of aggregate, the number of RFP or ubi-dsRED cells or the ubi-dsRED intensity, used was an automated fluorescence microscopic imaging system (ArrayScan® VTI HCS Reader) equipped with image analysis modules (BioApplications) that automatically convert images into numerical data for capturing changes in cell size, shape, intensity and other properties thereof in a culturing format. Protocols were written and designed for each purpose specifically according to the instruction manual. Prior to scanning, cells were initially fixed with 3% para-formaldehyde in PBS at 4°C for 1 hr and stained with 5 µg/ml Hoechst 33342 (Molecular Probes) for 30 min. Next, the cells were washed twice with PBS and subjected to the above-mentioned microscopic imaging system for a high-throughput analysis. To quantify the percentage of aggregates in the cells, in the first protocol, the number of EGFP-positive spots in each field was counted, and in the second protocol, the number of Hoechst-positive nuclei was counted in the same field. On the other hand, the quantification of RFP/ubi-dsRED cells could be achieved by a one scanning protocol. Scanning was usually performed in duplicate or triplicate using a 24-well plate under respective experiment conditions. Generally, 30,000 - 50,000 cells were scanned in each well (about 200 scanning fields), and quantification data of at least 200,000 cells in each experiment setting were generated by ArrayScan®.

### In vivo research of benzamil using R6/2 mice

HD mouse model R6/2 containing 145 CAG repeats was originally purchased from The Jackson Laboratory. Mice carrying 120 - 130 CAG repeats (confirmed by PCR) were selected for an *in vivo* test with benzamil. Benzamil dissolved in water was orally administered in an amount of 5 mg/kg (Ben-5) or 1 mg/kg (Ben-1) once a day six times a week (no administration one day a week) starting from 4 week-old until death. The body weight was measured once a week. For a clasping test, mice were suspended by the tail for 30 sec, clasping patterns were graded into the following levels according to the following criteria: 0: no clasping, 1: forelimb clasping only, 2: both forelimb and hindlimb clasping once or twice, 3: 3 times or more clasping for more than 5 sec of both forelimb and hindlimb. For a rota-rod test, mice were first placed on a rod rotating at a speed of 4 rpm, this speed was linearly increased up to 40 rpm over 300 seconds, and the speed of 40 rpm was maintained for 60 more sec. All the 5 - 12-week-old mice were subjected to the rota-rod test of the same rotational speeds. For the survival distribution, the number of days each mouse survived was recorded, and data collected for all treatments (mice survived for 13 weeks or longer; water, n=8; tg-Ben1, n=10; and tg-Ben5, n=9) were subjected to the Kaplan-Meier analysis followed by log-rank testing. For all the experiments involving mice, we received approval from the animal experiment committee of the RIKEN Brain Science Institute.

### Semi-quantitative PCR

Total RNA was extracted from the Neuro2a cells using an RNeasy® Mini Kit (QIAGEN) according to the instruction manual. To ensure complete removal of contaminating DNA, genomic DNA was digested with RNase-free DNase I (QIAGEN). The amount of total RNA was quantified based on the absorbance at 260 nm (GeneQuant, Amersham). Using SUPERSCRIPT™III RNase H⁻ Reverse Transcriptase (Invitrogen), 1 µg of RNA from each sample was transcribed into a cDNA. PCR was performed in a thermal cycler (BIO-RAD). For each primer, a cycle number that gives mid-log phase amplification was first determined, and the gene of interest was then amplified. The PCR product was separated by electrophoresis on 2.5% agarose gel. DNA images were captured by a UV transilluminator (TOYOBO CO., LTD.), and the band intensity was quantified by the ImageJ software (NCBI). Glyceraldehyde-3-phosphate-dehydrogenase (Gapdh) gene was amplified in each PCR experiment to serve as an internal loading control for a change in gene expression level. The PCR primers used in this research are as follows (synthesized by Sigma or Operon; all from 5' to 3'; F-forward; R-reverse): NHE1-F TACGGGGTCATCGCGGCTTT (SEQ ID NO: 1), -R ATGACGGTGATGATGGCGGT (SEQ ID NO: 2); NHE5-F CCACAGAAGAACCCACCCAG (SEQ ID NO: 3), -R AAAGACGGCCAACACGGCCA (SEQ ID NO: 4); NCX1-F TGACCGGAGCTGGCAACATC (SEQ ID NO: 5), -R TCTCGCTCACGTGAGTCACG (SEQ ID NO: 6); NCX2-F AGGCTGCCAAGGTTCCTACC (SEQ ID NO: 7), -R CCCACACCTGGACTACACCT (SEQ ID NO: 8); NCX3-F CTTCGTCACGGCTGCTTGGA (SEQ ID NO: 9), -R CTCAATGCCCTTAGGGTGGT (SEQ ID NO: 10); BNaC1-F CCGTCCCTGAGTCGCACTAA (SEQ ID NO: 11), -R CATGAAGGCAGCGCTGATGC (SEQ ID NO: 12); BNaC2-F TGCACGGTCTTGCCCACATC (SEQ ID NO: 13), -R GTCATGCCCTGCTCTGTCGT (SEQ ID NO: 14); egfp-F ATGGTGAGCAAGGGCGAGGA (SEQ ID NO: 15), -R CATGCCGAGAGTGATCCCGG (SEQ ID NO: 16); Gapdh-F ATTGTCAGCAATGCATCCTG (SEQ ID NO: 17), -R TTCAGCTCTGGGATGACCTTGCC (SEQ ID NO: 18). All the primer sequences were subjected to BLAST (NCBI), and they were highly specific.

### RNA interference

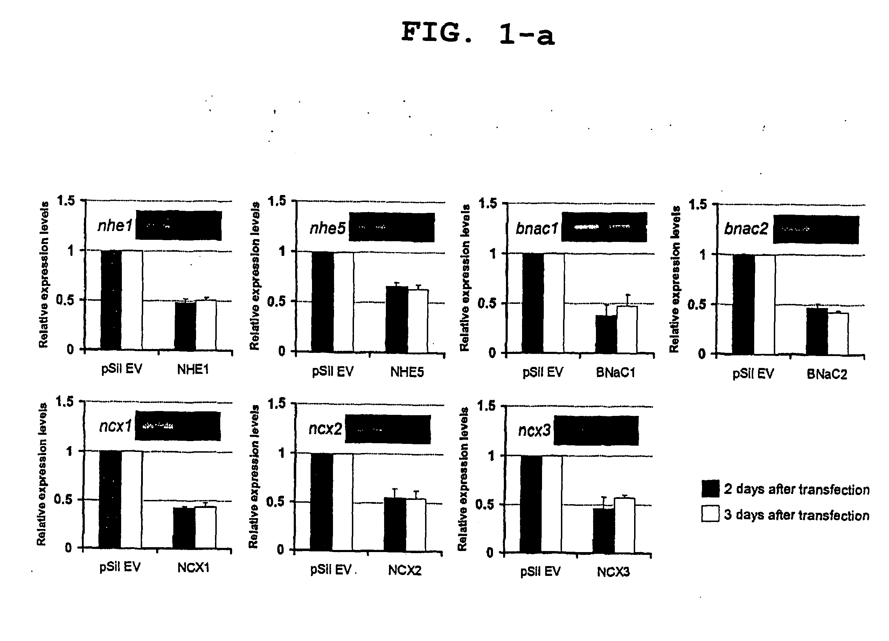

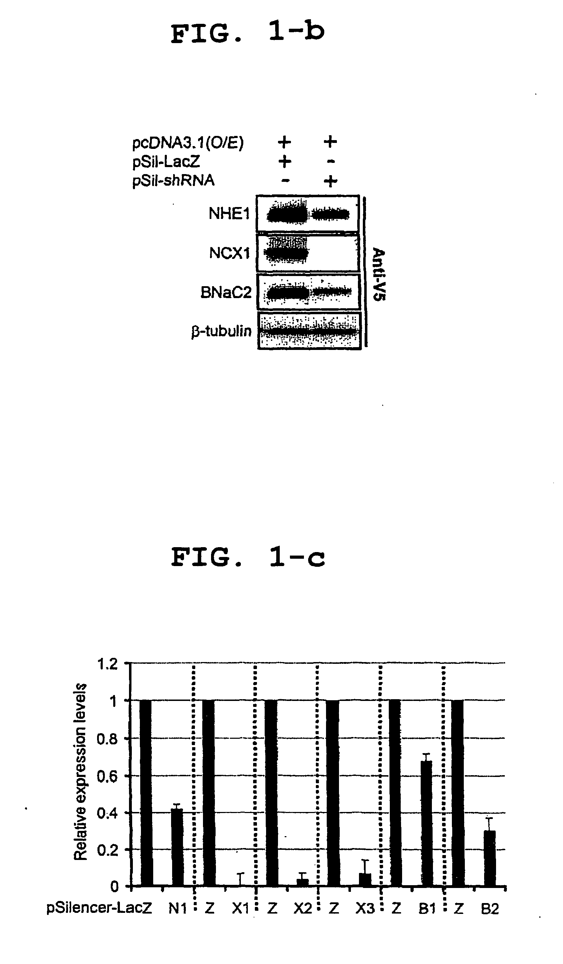

The sequences of 19 nucleotides were selected from the mRNA of each gene (Accession No.: NHE-1, U51112; NCX-1, NM_011406; NCX-2, NM_148946; NCX-3, NM_080440; BNaC1, BC038551; BNaC2, NM_009597). Since there was no complete mouse NHE-5 sequence at the time of the experiment, an RNAi experiment was performed, human NHE-5 sequence AF111173 was submitted to the mouse EST database (TGI) and a predicted mouse NHE-5 sequence with 91% homology was found by clustering 22 short EST sequences. The shRNA for mouse NHE5 was designed based on this MGI report using the siRNA Design Software (SDS) available online (http://i.cs.hku.hk/~sirna/software/sirna.php). This software recruited 12 siRNA designing softwares from major biotechnology companies, and ranked the popularity of each sequence to maximize the opportunity to select an effective siRNA sequence. The top 5 sequences from each search were subjected to BLAST (NCBI), and the specificity thereof was confirmed prior to ordering. Sense and antisense templates with a hairpin backbone were each synthesized (Operon) individually, annealed, and ligated into a pSilencer 1.0 vector driven by U6 promoter according to the instruction manual (Ambion). All the plasmids containing a shRNA producing sequence was sequence-verified. The shRNA producing sequence used in this research are as follows (those that were tested for effectiveness; all from 5' to 3'): NHE1-1:
GGACAAGCTCAACCGTTTT (SEQ ID NO: 19); NHE1-2:
GGAGGAAGAGATCCGCAAA (SEQ ID NO: 20); NHE5-1:
GAGGAGTGATTACCACAAA (SEQ ID NO: 21); NHE5-2:
AGGGCGTCGCCTCCCTGTT (SEQ ID NO: 22); NCX1-1:
CTGGGTCTGATTATGAATT (SEQ ID NO: 23); NCX1-2:
AGAGTTGGCATCATTGATG (SEQ ID NO: 24); NCX2-1:
CTTCAGGTCAAGATAGTGG (SEQ ID NO: 25); NCX2-2:
GAATGGAGACAAGAAGATA (SEQ ID NO: 26); NCX3-1:
TAATTGATGATAAGGCGTA (SEQ ID NO: 27); NCX3-2:
ACAATTCGGGTATGGAATG (SEQ ID NO: 28); BNaC1/ASIC2-1:
ACAGCAATGAACACCAAAG (SEQ ID NO: 29); BNaC1/ASIC2-2:
CCCAAGGACAGCAATGAAC (SEQ ID NO: 30); BNaC2/ASIC1-1:
TCAATGAGTTTCGCTTTAG (SEQ ID NO: 31); BNaC2/ASIC1-2:
GAGCGTGTGCAGTACTACT (SEQ ID NO: 32); BNaC2/ASIC1-3:
AGTTCAACAAATCTGAACA (SEQ ID NO: 33).
LacZ: TCTATCGTGCGGTGGTTGA (SEQ ID NO: 34)
(as a negative control for transfection and silencing). A plasmid containing an shRNA insert was transfected into Neuro2a cells with Lipofectamine 2000. Silencing was allowed to proceed for 2 days, total RNA was collected, and semi-quantitative PCR was performed as mentioned above (the silencing effect of our shRNA at the transcriptional level is shown in Fig. 1a). To show that the silencing effect also occurred at the post-translational level, the expression vector of each channel protein and the corresponding shRNA silencing vector thereof were co-transfected. LacZ shRNA was used as a negative control. Fig. 1b shows a significant downregulation revealed by an anti-V5 antibody.

### Western blot

Cells for use as a test subject were rinsed with cold PBS and lysed in a suitable amount of a lysis buffer (8.6% sucrose, 1 mM EDTA, 1 mM sodium orthovanadate (Na₃VO₄), 10 mM sodium fluoride (NaF), 50 mM Tris, 0.038% EGTA, 1% Triton X-100 and Complete™). Thereafter, a protein lysate was sonicated briefly. The protein concentration was measured with a Bicinchoninic Acid (BCA) Protein Assay Kit (Pierce) according to the instruction manual. Next, a suitable amount of the protein lysate was mixed with 4 X SDS gel-loading buffer (200 mM Tris-HCl, pH 6.8, 400 mM DTT, 8% SDS, 40% glycerol and a suitable amount of bromophenol blue), boiled at 100°C for 5 min, and separated on 5 - 20% gradient polyacrylamide gel (ATTO). Thereafter, the protein was transferred onto a PVDF membrane (0.22 µm, Schleicher & Schuell), and the membrane was blocked for 1 hr with 5% non-fat milk in TBS-T (10 mM Tris, pH 7.5, 50 mM NaCl and 0.1% Tween-20, Sigma). Then, the blot was incubated with suitably diluted primary antibodies and HRP-conjugated secondary antibodies (both at room temperature for 1 hr, extensively washed with TBS-T between and after each incubation). Eventually, the blot was developed with ECL solution and exposed onto a Hyperfilm™ MP (Amersham Biosciences).

### Chase experiment

To determine whether soluble tNhtt-polyQ is degraded faster in the presence of benzamil, a chase experiment was performed, because inducible Neuro2a cells that express tNhtt-60Q were selected. Majority of the tNhtt-60Q remained soluble, and only few aggregates were observed one day after incubation. Concisely stated, Neuro2a cells were first differentiated, and induced to express hrtNhtt-60Q for 24 hours. Ponasterone A was removed thereafter (with dbcAMP maintaining the differentiation status), and the cells were incubated with water (control) or 50 µM benzamil (Day 0) for 4 days (Day 4). The medium was replaced with a fresh medium containing benzamil in the same concentration every 2 days, and cells were collected once a day until Day 4. Cells were then lysed, and expression of soluble tNhtt-60Q was analyzed by Western blot with an anti-huntingtin antibody (anti-em48).

### Filter trap assay

A filter trap assay was performed with a Hybri-Dot manifold (BIO-RAD) and a cellulose acetate membrane filter having a pore size of 0.2 µM (Advantec). Concisely stated, cells ready for analysis were lysed in the same lysis buffer as that used for the Western blot and sonicated briefly, and the protein concentration were quantified according to the above-mentioned BCA method. For a brain sample, brain tissues were initially homogenized 10 strokes at 1500 rpm in a 5 cm³ glass tube with 1 ml homogenization buffer (50 mM Tris-HCl, pH 7.4, 150 mM NaCl, 1% Triton X-100, 1 mM PMSF, and a complete protease inhibitor cocktail). Then, brain lysate was sonicated briefly, and the concentration was measured. The same amount of proteins from respective experiment conditions were each diluted by 2% SDS in PBS to 100 µl, and applied onto the membrane. The wells were washed twice with 2% SDS/PBS and vacuum-suctioned for 20 min, thereby aggregate insoluble in SDS being completely and securely trapped. The membranes were then blocked with 5% skim milk and Western blotting was proceeded.

### Statistical analysis

For comparing two samples, an unpaired student's t-test was used. For comparing multiple samples, a one-way ANOVA Fisher's test followed by a Tukey's HSD test was used with a confidence level of 95%. For measuring the significance between different populations, a two-sample Kolmogorov-Smirnov test was used. For survival rate, a survival distribution curve was plotted with the Kaplan-Meier method, and the survival rate was then calculated with the log-rank testing (all the data were generated with the XLSTAT software). For all the statistical analysis, difference between the comparisons was considered significant when p<0.05.

### Others

The intensity of immunoblot band was scanned with the EPSON ES-8000 scanner and then quantified with the ImageJ (version 1.32j, NCBI), or captured with the FUJIFILM LAS-1000 plus system and analyzed using an image analyzing software. A value obtained from one data point was divided by a value obtained for the internal control thereof (β-tubulin) and set to 100%. The ratios obtained for other data points were compared in the same manner, and expressed as relative ratio of change.

### Results

### Amiloride and benzamil decreased tNhtt-polyQ aggregate in a dose-dependent manner.

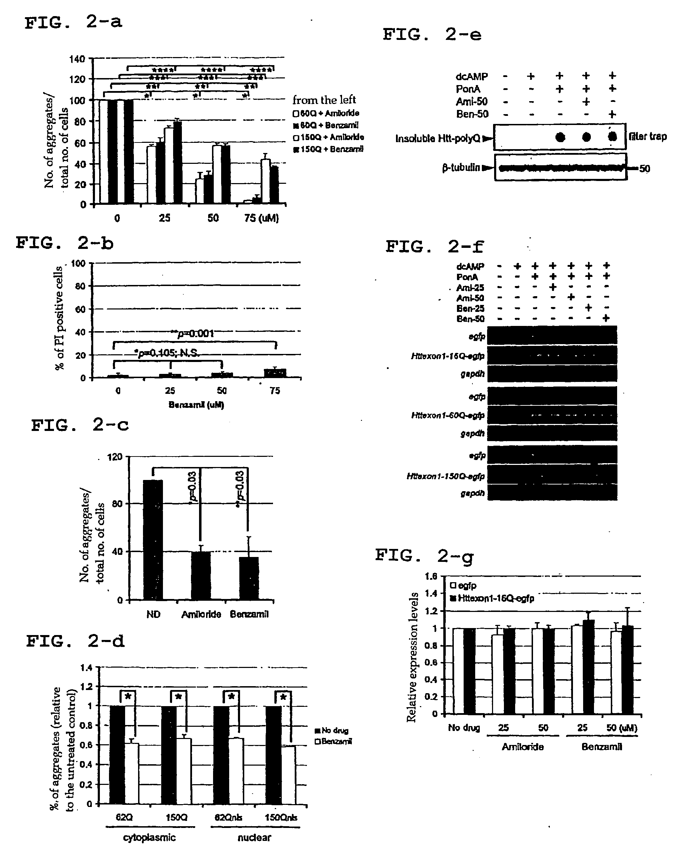

To test whether amiloride (Ami) and a derivative thereof, i.e., benzamil (Ben), have any influence on the protein aggregate formed by tNhtt-polyQ, first, employed was a stable mouse neuroblastoma Neuro2a cell system in which expression of tNhtt-polyQ-EGFP (tNhtt, truncated N-terminal Huntingtin; polyQ, 60 or 150 polyglutamine repeats) is induced by 1 µM ponasterone A and which that can differentiate into neuronal phenotype cells in response to treatment with 5 mM N6,2'-O-dibutyryladenosine-3',5'-cyclic monophosphate sodium salt (dbcAMP) [Wang, G.H. et al. Neuroreport 10, 2435-8 (1999)]. It was found that both Ami and Ben decreased tNhtt-60Q-EGFP and tNhtt-150Q-EGFP cytoplasmic aggregates without significant toxicity up to 50 µM (Fig. 2b, *p=0.105 and **p=0.001; 50 µM or less was used throughout the research for the cell system) similarly in a dose-dependent manner (Fig. 2a, for treated group versus untreated group p<0.0001;* and **p=0.019;*** and ****p<0.0001). Moreover, Ami and Ben significantly decreased tNhtt-150Qnls-EGFP nuclear aggregates in the same inducible system (Fig. 2c, * and **p=0.03). To eliminate the possibility of Ami and Ben decreasing aggregates by interfering with the ponasterone A-inducible system, native Neuro2a cells were transfected with an expression plasmid having 62 or 150 polyglutamine repeats in the presence or absence of a nuclear localization signal peptide (Fig. 2d). As with the results obtained in the inducible system, both drugs significantly decreased cytoplasmic and nuclear aggregates by 40% (*p=0.008; amiloride, data not shown). In addition, it was shown that, in a filter trap assay, Ami and Ben reduced the amount of aggregate trapped on a cellulose acetate membrane loaded with the same amount of protein (Fig. 2e). It shows that it is not that Ami and Ben decrease aggregation by masking the EGFP fluorescence that can be captured by the ArrayScan® VTI HCS Reader. Since semi-quantitative PCR showed that Ami and Ben did not influence the expression level of tNhtt-16/60/150Q (images in Fig. 2f and quantification in Fig. 2g), the data obtained above suggest that these drugs decrease tNhtt-polyQ aggregation through the post-transcriptional mechanism.

### Benzamil ameliorates HD pathology in R6/2 mice.

Because of the significant and remarkable decrease of tNhtt-polyQ aggregation in the cell system by amiloride and benzamil, we decided to investigate whether or not the same disaggregating effect can be observed in an animal model of HD. Although the mechanism of the decrease of tNhtt-poly aggregation by amiloride and benzamil was not clear [Cuthbert, A.W. & Edwardson, J.M. J Pharm Pharmacol 31, 382-6 (1979)], since benzamil has higher affinity with its target protein compared with amiloride, benzamil was first selected in the research using mice. To investigate the advantageous effect of benzamil *in vivo*, employed was an R6/2 (transgenic, tg) mouse model in which progressive HD pathology are well characterized and which is widely used in a pre-clinical test of a drug [Mangiarini, L., et al. Cell 87, 493-506 (1996)]. Ben in an amount of 1 mg/kg (Ben 1) or 5 mg/kg (Ben 5) was orally administered 6 times a week to R6/2 mice (having 120 - 130 polyQ repeats), and for a control for comparison, water was used (Fig. 3). The limb clasping posture of the tg mice was remarkably ameliorated in 8-week-old mice that had been given Ben (tg-Ben 1 and tg-Ben 5) (Fig. 3a, * and **p<0.0001). When all the control mice (transgenic mice given water) reached level 3 of clasping posture, better amelioration in clasping posture was obtained by 1 mg/kg of benzamil, and the advantageous effect was continuously observed even at 12 week-old. (Fig. 3a, ***p=0.009). To give a more objective evaluation showing that the motor deficit in transgenic R6/2 mice can be ameliorated by benzamil, a rota-rod experiment was performed using 1 mg/kg benzamil-administered wild-type mice and transgenic mice (Fig. 3b). Although there was no substantial difference among the wild-type group mice, tg-Ben1 greatly prolonged the period of time the transgenic mice stayed on the rota-rod, and this fact further suggests that the motor deficit of the R6/2 mice was remarkably reduced (Fig. 3b, *p=0.0015 and **p=0.042).

Next, whether or not benzamil ameliorates HD pathology in the brain was investigated (Figs. 3c and 3d). From the wild-type, tg-water, tg-Ben1 and tg-Ben5, whole brain lysates were prepared. As shown in Fig. 3c, a significant decrease of aggregation was observed with the Ben1- or Ben5-treated mice. The quantification of spot intensity on the filter trap membrane revealed that the aggregates detected by the anti-huntingtin (em48) antibody was decreased in both 8-week-old and 12-week-old mice by more than 30% (Fig. 3d, 8 weeks water vs. Ben, p=0.003; 12 weeks water vs. Ben, p=0.04;*p=0.009;**p=0.004;*** and ****p=0.007). Western blotting using the same protein lysate showed the equal loading as that revealed by the β-tubulin antibody (data not shown). Since benzamil could be detected and quantified in the cerebrospinal fluid (CSF) of the experimental rats, we assumed that benzamil exerted a direct effect in the brain tissue to decrease the amount of polyQ aggregations (Fig. 4). Accompanied by the amelioration of the pathology in the brain and the motor deficits, a significant increase in body weight was observed in tg-Ben1 and tg-Ben5 after 10 week-old, (Fig. 3e, *p=0.034 and **p=0.044). More importantly, Ben1 significantly increased the survival rate of R6/2 mice analyzed with the Kaplan-Meier method followed by log-rank test (Fig. 3f). The average survival period of the tg-water was 114.625±2.44 days, and the average survival period of the tg-Ben1 was 126.1±4.001 days (tg-water vs. tg-Ben1, p=0.024). Although tg-Ben5 did not show an overall increase in survival rate, some of them survived much longer than the mice treated with water only (line with black circles in Fig. 3f). If summarized, based on the data collected from the R6/2 mice, we concluded that benzamil ameliorates the motor deficits and pathology and increases the survival rate in a mammalian model of HD.

### Benzamil enhances proteasome activity globally.

After we were convinced that benzamil ameliorates the HD symptoms in both *in vitro* (Fig. 2) and *in vivo* (Fig. 3) models, we investigated the mechanism of benzamil for reducing tNhtt-polyQ aggregation. Given that Ami and Ben do not influence the expressional level of tNhtt-polyQ at the transcriptional level (Fig. 2f and Fig. 2g), we were prompted to conclude from the observation of a constant and consistent decrease in soluble huntingtin expression, that the degradation system can be enhanced under Ami- or Ben-treated conditions. As shown in Fig. 5a and as quantified in Fig. 5b, a significant decrease of soluble huntingtin was consistently observed, in the Neuro2a cells treated with 50 µM Ami and Ben (Fig. 5b, *p=0.008, amiloride, data not shown). Moreover, the images captured by the ArrayScan® VTI HCS Reader repetitively showed that there was a significant decrease in the percentage of GFP-positive cells and in the GFP intensity in both the benzamil-treated inducible system and temporary system (Fig. 5c, *p<0.0001). First, the ubiquitin proteasome system (UPS) in the cells treated with benzamil was examined. To make the examination possible, an expression construct that expresses the ubiquitin-tagged Discosoma Red fluorescent protein (dsRED) (ubi-dsRED) was used. It has been shown that the number of ubi-dsRED cells and the intensity of ubi-dsRED fluorescence directly reflect the *in vivo* proteasome activity and that polyQ blocks UPS in both C.elegans and an *in vitro* HD cell model [Khan, L.A. et al. J Neurochem 98, 576-87 (2006)]. Using ubi-dsRED, *in vivo* proteasome activity in the presence of benzamil was examined.

To examine whether or not benzamil can enhance *in vivo* proteasome activity, first, Neuro2a cells were transfected with a very small amount of ubi-dsRED construct, and then proteasome inhibitor, MG-132 and benzamil of various concentrations were added. Benzamil could alleviate proteasome inhibition caused by MG-132 in a dose-dependent manner (Fig. 5d and Fig. 5e). The number of ubi-dsRED cells was normalized to that when not treated by benzamil, and a gradual decrease in ubi-dsRED cells was then observed as the benzamil concentration increased (Fig. 5d, MG-132 vs. Ben, p<0.0001;*p=0.019). Although an increase in MG-132 concentration led to ubi-dsRED cell accumulation, an increase in benzamil concentration alleviated the accumulation as shown by Western blotting (Fig. 5e). These data suggest that benzamil has an ability to globally enhance proteasome activity, and this is one of the mechanisms of benzamil that decrease tNhtt-polyQ aggregation *in vitro* and *in vivo.*

### Benzamil enhances proteasome activity in polyQ-bearing cells, and enhances the degradation of soluble tNhtt-polyQ.

Whether or not benzamil enhances proteasome activity in cells bearing polyQ repeats was investigated. First, EGFP, 17Q-EGFP or 150Q-EGF was co-transfected with Pubi-dsRED in a ratio of 3:1 into Neuro2a cells, and the intensity of ubi-dsRED fluorescence in EGFP-positive cells were quantified. As shown in Fig. 6a, the enhancement dependent on the length of polyQ was observed in the intensity of ubi-dsRED fluorescence (distributions of EGFP vs. 17Q-EGFP, EGFP vs. 17Q-EGFP or 17Q-EGFP vs. 150Q-EGFP were all significantly different; p<0.0001, analyzed by the two-sample Kolmogorov-Smirnov test). These data support that tNhtt-polyQ can block UPS [Bennett, E.J., Bence, N.F., Jayakumar, R. & Kopito, R.R. Mol Cell 17, 351-65 (2005); Bence, N.F., Sampat, R.M. & Kopito, R.R. Science 292, 1552-5 (2001)] and the ubi-dsRED construct is an effective tool that reflects the proteasome activity in polyQ-bearing cells [Khan, L.A. et al. J Neurochem 98, 576-87 (2006)].

Using monomeric RFP (mRFP) [Machida, Y. et al. Biochem Biophys Res Commun 343, 190-7 (2006)] as an internal control, first, investigated was the proteasome activity in cells transiently co-transfected with tNhtt-polyQ and ubi-dsRED, in the presence or absence of benzamil. When RFP and polyQ were co-transfected, complete co-localization of aggregates and polyQ was observed, and benzamil did not substantially influence this co-localization (Fig. 6b, A and B; and, Fig. 6c, RFP). However, while tNhtt-polyQ and ubi-dsRED maintained complete co-localization (Fig. 6b, C - F), in a large population of polyQ-bearing cells, co-localization with ubi-dsRED was no longer observed due to benzamil addition (Fig. 6b G - K and Fig. 6c, Ubi-dsRED; *p<0.0001, water vs. polyQ). Consistent with the decrease in co-localization of polyQ and ubi-dsRED, the number of ubi-dsRED cells in the polyQ-bearing population (Fig. 6d) and the average ubi-dsRED intensity in the polyQ-bearing cells (Fig. 6e) were also significantly decreased. Western blotting with an anti-RFP antibody also revealed that while addition of 10 µM lactacystin greatly increased accumulation of ubi-dsRED cells, accumulation of ubi-dsRED cells was mostly absent in benzamil-treated cells (Fig. 6f). The same experiment was performed using RFP, and similar results were not observed (data not shown). Data presented herein strongly suggest that proteasome activity in polyQ-bearing cell is remarkably enhanced.

Given that *in vivo* proteasome activity is greatly enhanced in the presence of benzamil, degradation of soluble tNhtt-polyQ is expected to be enhanced. To examine whether or not the degradation rate of soluble tNhtt-polyQ is faster with benzamil treatment, a chase experiment was performed in which expression of tNhtt-60Q was induced for 1 day, then the induction was terminated by adding benzamil (designated as Day 0), and a protein lysate was collected until Day 4 (Fig. 6g). It was shown that the benzamil treatment suppressed the initial increase in tNhtt-60Q expression (Day 1. probably caused by the residual amount of ponasterone A) and thereafter the degradation rate was similar to that obtained with the water control (from Day 2 to Day 4). There was a significant decrease in the absolute amount of soluble tNhtt-polyQ at Day 3 and Day 4 in the presence of benzamil (*p=0.037 and ^{#}p=0.039, respectively), and the half-life of soluble tNhtt-60Q was determined as being 5.294±0.52 days for the water control and 3.846±0.32 days for benzamil. The same experiment was also performed for tNhtt-16Q. There was no significant difference in degradation rate between water and the benzamil treatment (data not shown).

### UPS, but not autophagy, is the major pathway through which benzamil recruits tNhtt-polyQ clearance.

In addition to the UPS system, autophagic degradation of polyQ draws much attention recently in the field of neurodegeneration demonstrated by Rubinsztein's group [Williams, A. et al. Curr Top Dev Biol 76, 89-101 (2006)]. The macroautophagy has been demonstrated to be one of the important pathways to decrease polyQ aggregation and toxicity in the HD model [Ravikumar, B. et al. Nat Genet 36, 585-95 (2004)]. From the evidence obtained hitherto, it was not possible to exclude the possibility that autophagy, in company with UPS or secondary to UPS, is not involved in the degradation of soluble or aggregated tNhtt-polyQ by benzamil. To investigate whether or not other degradation pathways play a major role in the degradation of tNhtt-polyQ by benzamil, first, the proteasome pathway was blocked by a specific proteasome inhibitor, i.e., lactacystin or MG-132, and the number of tNhtt-polyQ aggregates under these experiment conditions was quantified. The blocking of proteasome by lactacystin or MG-132 attenuated the disaggregating effect exerted by benzamil, although the attenuation was not complete compared with the addition of the proteasome inhibitor itself (Fig. 7a, *p=0.006;**, ***, ****p<0.0001). It appears that complete abolishment could be achieved only when the proteasome pathway and the autophagic degradation pathway (by 5 mM 3-methyladenine) were both simultaneously blocked (Fig. 7b, *p=0.09, not significant). It was also revealed that by probing with an anti-ubiquitin antibody a lysate prepared from cells treated with lactacystin or MG-132, both proteasome inhibitors almost completely abolished the disaggregating effect of Ami or Ben, and this is consistent with the data quantified by the ArrayScan® (Fig. 7c). To further exclude the possibility that macroautophagy plays an important role in the benzamil-mediated tNhtt-polyQ clearance, used was a stable mouse embryonic fibroblast (MEF) cell line in which expression of autophagy gene 5 (Atg5) can be readily manipulated by addition of tetracycline. It has been shown that the Atg12-Atg5 conjugate can be completely suppressed by adding 1 ng/ml of doxycycline for 4 days [Hosokawa, N., Hara, Y. & Mizushima, N. FEBS Lett 580. 2623-9 (2006)]. Truncated Nhtt-polyQ was transfected into the MEF that had been treated with doxycycline for 5 days, and then benzamil was added for 18 hr. The fact that the presence or absence of Atg5 did not substantially influence the degree of aggregation further suggests that macroautophagy is not required for tNhtt-polyQ clearance caused by benzamil (Fig. 7d, *p>0.05, not significant).

### ASIC/BNaC and NCX RNAi increase proteasome activity and decrease tNhtt-polyQ aggregation.

Since it has been shown that benzamil may decrease tNhtt-polyQ aggregation by promoting *in vivo* proteasome activity, we investigated the basic mechanism of Ami and Ben for increasing proteasome activity. Ami and Ben have several common target proteins such as NHE, ASIC/BNaC, NCX and the like. We investigated a possibility that Ami and Ben increase proteasome activity by inhibiting these channel proteins.

For this purpose, we attempted to mimic the pharmacological blocking of NHE, ASIC/BNaC and NCX by reducing the expression of these channel proteins by RNA interference (RNAi). At least 5 different shRNAs were designed that target a variety of regions in mRNA, and cloned into pSilencer 1.0 vector driven by U6 promoter. Fig. 1 shows that the expression level of all these genes was significantly blocked by the corresponding shRNA at both the transcriptional level (endogenous level, Fig. 1a) and the post-translational level (exogenously induced, Fig. 1b and 1c). As the comparison controls for all the RNAi experiment, LacZ shRNA expression construct was also prepared.

First, we investigated whether or not the blocking of these channel proteins influences tNhtt-polyQ aggregation similar to the effect of Ami or Ben. Inducible Neuro2a cells (150Q) were initially transfected for 2 days with shRNAs corresponding to each gene and induced for 1 day so as to express tNhtt-150Q. By inhibiting the expression of ASIC/BNaC (Fig. 8a, *p=0.003; **p=0.006 and ***p<0.0001) or NCX (Fig. 8b, *p<0.0001), the percentage of aggregation was decreased, and by blocking both ASIC/BNaC and NCX, the amount of aggregates in 60Q cells and 150Q cells was remarkably decreased (Fig. 8c, *p<0.0001). Interestingly, when an NHE isoform or both NHE1 and NHE5 were blocked, no significant decrease of aggregation was obtained. This suggests that NHE may not be actively involved in the process of tNhtt-polyQ aggregation (Fig. 8d). Next, proteasome activity in cells in which the expression of channel protein was suppressed with ubi-dsRED construct was investigated. Compared with the LacZ shRNA control, the number of ubi-dsRED cells was significantly decreased when the expression of NCX, ASIC/BNaC, or both NCX and ASIC/BNaC was blocked. This is similar to the results shown in Fig. 6 (Fig. 8e; *p<0.0001). These data strongly suggest that the enhancement of *in vivo* proteasome activity by amiloride or benzamil, although a possibility of a direct influence on the UPS system cannot be excluded, can be mediated by blocking of ASIC/BNaC and/or NCX expressed on the plasma membrane.

### Sequence Listing Free Text

SEQ ID NOs: 1 - 18: primer
SEQ ID NOs: 19 - 34: shRNA producing sequence

## Claims

1. A compound represented by the formula (I) 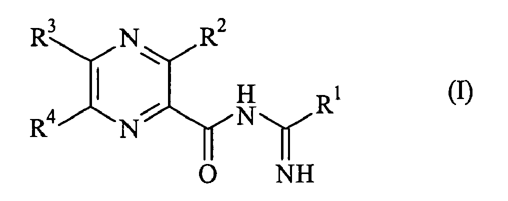 wherein R¹, R² and R³ are each independently an optionally substituted amino group, and R⁴ is halogen, or a pharmacologically acceptable salt thereof for use in a method for the prophylaxis or treatment of a neurodegenerative disease.

2. A compound according to claim 1, wherein R¹ is an amino group substituted by an optionally substituted lower alkyl group or an optionally substituted aryl group.

3. A compound according to claim 2, wherein R¹ is an amino group substituted by an unsubstituted aryl group or a lower alkyl group substituted by an aryl group, and R² and R³ are each an unsubstituted amino group.

4. A compound according to any of claims 1 to 3, wherein R⁴ is chlorine.

5. A compound according to claim 1, wherein the compound represented by the formula (I) is amiloride, benzamil or phenamil.

6. A compound according to any of claims 1 to 5, wherein the neurodegenerative disease is selected from the group consisting of polyglutamine disease, Alzheimer's disease, Parkinson's disease and amyotropic lateral sclerosis.

7. A compound according to any of claims 1 to 5, wherein the neurodegenerative disease is Huntington's disease.

8. A compound represented by the formula (I) as defined in any one of claims 1 to 5 or a pharmacologically acceptable salt thereof for use in a method for enhancing a proteasome activity.

9. Use of a compound represented by the formula (I) as defined in any one of claims 1 to 5 or a pharmacologically acceptable salt thereof for the production of an agent for the prophylaxis or treatment of a neurodegenerative disease.

10. Use of a compound represented by the formula (I) as defined in any one of claims 1 to 5 or a pharmacologically acceptable salt thereof for the production of a proteasome activity enhancer.
